Europäisches Patentamt

European Patent Office

Office européen des brevets

(19)

(11) Publication number : **0 393 825 B1**

# (12) EUROPEAN PATENT SPECIFICATION

(45) Date of publication of patent specification :
**15.06.94 Bulletin 94/24**

(21) Application number : **90302420.6**

(22) Date of filing : **07.03.90**

(51) Int. Cl.⁵ : **C08L 1/02,** C08L 3/02,
C08J 5/04, // (C08L1/02,
5:08)

(54) **Novel water-absorptive composite material and method for the preparation thereof.**

(30) Priority : **21.04.89 JP 102951/89**

(43) Date of publication of application :
**24.10.90 Bulletin 90/43**

(45) Publication of the grant of the patent :
**15.06.94 Bulletin 94/24**

(84) Designated Contracting States :
**BE DE ES FR GB IT NL**

(56) References cited :
**EP-A- 0 323 732**
**CH-A- 657 370**
**GB-A- 2 050 459**
**US-A- 4 099 976**
**PATENT ABSTRACTS OF JAPAN, vol. 5, no. 12
(C-40)[684], 24th January 1981; & JP-A-55 141
171 (KURARAY K.K.) 04-11-1980**
**PATENT ABSTRACTS OF JAPAN, vol. 12, no.
270 (C-515)[3117], 27th July 1988; & JP-A-63 49
212 (TOYO ROSHI K.K.) 02-03-1988**
**R.L. Whistler, Starch: Chemistry and Technology, 2nd ed., Academic Press, Inc., New York
1984, pages 285-286**

(73) Proprietor : **Japan as represented by
Director-General, Agency of Industrial
Science and Technology
3-1, Kasumigaseki 1-chome
Chiyoda-ku
Tokyo 100 (JP)**

(72) Inventor : **Hosokawa, Jun
1095-239, Yashimahigashi-machi
Takamatsu-shi, Kagawa-ken (JP)**
Inventor : **Nishiyama, Masashi
1023, Motodai-cho
Kan-onji-shi, Kagawa-ken (JP)**
Inventor : **Kubo, Takamasa
1-30, Yashima-jutaki,
1403, Yashimanishi-machi
Takamatsu-shi, Kagawa-ken (JP)**
Inventor : **Yoshihara, Kazutoshi
2-201, Saiho-cho-jutaku,
14-6, Saiho-cho 1-chome
Takamatsu-shi, Kagawa-ken (JP)**

(74) Representative : **Baverstock, Michael George
Douglas et al
BOULT, WADE & TENNANT
27 Furnival Street
London, EC4A 1PQ (GB)**

## Description

The present invention relates to a novel composite material in the form of a shaped body having water-absorptivity but free from disintegration in water. More particularly, the present invention relates to a shaped body of a composite material basically formed of cellulose fibers and chitosan and having biodegradability, for example, in soil. The invention also relates to a method for the preparation of such a composite material.

The composite material of the invention is useful in a wide variety of applications, mainly but not limitatively, in the form of a sheet used, for example, as a sanitary absorbent material, sheet material for agricultural uses, temporary pots for growing young garden trees and seedlings and the like. The services expected for the sheet materials in the above mentioned applications are mostly of a temporary nature so that it is desirable that the sheet material can be spontaneously decomposed after use or after lapse of the service season without leaving any decomposition products which may cause a problem in environmental pollution.

The most serious problem in the widely prevailing applications of synthetic resin- and fiber-made articles is that synthetic resin in general is highly resistant to attack from microorganisms so that debris of the articles remain in the soil or in water semipermanently so as to cause a problem of environmental pollution as a counterpart of the advantageous durability of the articles. Incineration treatment as a means of waste disposal is also not free from some problems when waste materials are synthetic resin-made. In view of these problems, it is highly and eagerly desired to develop a polymeric material suitable for shaping of various articles having moderate durability but capable of being rapidly decomposed microbiologically when the article has finished its service without causing problems in environmental pollution.

Many of the naturally occurring polymeric materials of botanical origin such as cellulosic pulp as a typical carbohydrate polymer may meet the above mentioned requirements of biodegradability to some extent. Cellulosic materials, however, have a limitation in respect of the shaping method applicable thereto and it is a difficult matter to prepare a precision-shaped article from a cellulosic material. Moreover, an article shaped from a cellulosic material is defective in respect of durability and stability, in particular, in water. Starch is another typical carbohydrate polymer but is highly swellable in water so that a shaped body made from starch is readily disintegrated in water unless the shaped article is subjected to a crosslinking treatment by using formaldehyde and the like as a crosslinking agent while even a trace amount of formaldehyde remaining in the shaped articles may cause a serious problem to human health.

It is known, on the other hand, that some naturally occurring polymeric materials of animal origin can be used as a material of shaped articles including, for example, chitosan. A problem in the use of chitosan is that, while chitosan is obtained in the form of a salt, chitosan salts cannot be shaped, for example, into films by mere drying without the troublesome procedure of fixing by an alkali treatment.

Two of the inventors have previously proposed in JP-A-63-237599 a biodegradable composite polymeric material made from cellulosic fibers and chitosan. The polymer proposed, however, is poorly water-absorptive so that it is also desirable to impart such a biodegradable composite material with increased water-absorptivity, for example, in application to sanitary absorbent materials.

An object of the present invention accordingly is to provide a shaped article of a novel biodegradable composite polymeric material from naturally occurring polymeric materials having adequate water-adsorptivity yet not to cause disintegration in water though to be swellable to some extent.

Thus, the invention provides a novel shaped body of a water-adsorptivity and biodegradable polymeric composite material which comprises:

(A) 100 parts by weight of fine cellulose fibers having a freeness of 50 ml or less of Canadian standard freeness;

(B) from 5 to 100 parts by weight of chitosan; and

(C) from 10 to 300 parts by weight of gelatinised starch.

The above defined cellulose fiber-based water-adsorptive and biodegradable polymeric composite material is prepared by a method which comprises the steps of:

(a) blending 100 parts by weight of fine cellulose fibers having a freeness of 50 ml or less of Canadian standard freeness, an aqueous solution of a chitosan salt in an amount from 5 to 100 parts by weight as chitosan and from 10 to 300 parts by weight of gelatinised starch to give an aqueous slurry;

(b) shaping the aqueous slurry of the compound into a form; and

(c) drying the thus shaped form of the compound by heating at a temperature in the range from 50 to 200°C.

As is described above, the essential components in the inventive composite material include cellulose fibers, chitosan and gelatinised starch. These materials are each a naturally occurring polymeric material and can be rapidly decomposed microbiologically in the soil or in water including sea water and river water without the problem of environmental pollution due to decomposition products. When either one of these materials alone is shaped and dried into a shaped body, however, it can hardly be expected that such a shaped body retains the dry form or mechanical strength under a watery environment encountered in water or in moistened soil. This is also the case with shaped articles prepared from a combination of cellulose fibers and starch or a chitosan salt and starch. Although a composite material of cellulose fibers and a chitosan salt may have improved water resistance, such a composite material is poor in water absorptivity so that such a composite material cannot be used in applications where water-absorptivity of the material is desirable. On the contrary, it has been unexpectedly discovered that a ternary composite material of cellulose fibers, chitosan and gelatinised starch has remarkably improved water absorptivity without substantial decrease in water resistance and retaining shape in water. The biodegradability of the inventive composite material can be controlled by suitably selecting the proportion of the components and the conditions for shaping.

The type of the cellulose fibers used as the component (A) in the inventive composite material is not particularly limitative including so-called lignocellulose, pectocellulose, bacteria cellulose and the like. It is important that the cellulose fibers have high fineness as imparted by any known method such as beating in a beater conventional in the paper-making industry to have a specified Canadian standard freeness of 50 ml or less.

Starch as the component (B) of the inventive composite material is a carbohydrate polymer of low cost as obtained from various kinds of plants accumulating starch as a nutritive source. The type or origin of the starch is not particularly limitative including potato, Indian corn, wheat, rice and the like.

Chitosan as the component (C) in the inventive composite material is a nitrogen-containing polymeric material derived from chitinous material occurring in nature as a constituent of the shell of crustacean animals and certain fungal bodies. Specifically, chitosan is a deacetylation product of chitin with a relatively high degree of deacetylation. In the method of the present invention, it is desirable that the degree of deacetylation is at least 40% in order that the chitosan is soluble in an aqueous medium acidified, for example, with acetic acid forming an acetate.

In the preparation of the inventive composite material, 100 parts by weight of fine cellulose fibers are blended with 5 to 100 parts by weight of the chitosan salt, e.g., acetate, in the form of an aqueous solution and then admixed with an aqueous solution of gelatinised starch prepared in advance by heating a dispersion of starch in water so as to give an aqueous slurry. The amount of the gelatinised starch is in the range from 50 to 300 parts by weight per 100 parts by weight of the cellulose fibers.

The above mentioned order of addition of the chitosan salt and the gelatinised starch can of course be reversed. It is optional as required to mix the aqueous slurry with plasticizers, fillers, coloring agents and the like. The aqueous slurry of the essential and optional components are then shaped and dried and heat-treated at a temperature in the range from 50 to 200 °C so that the blend is converted into a composite material shaped in a desired form such as a sheet or film. The length of time for this heat treatment should not be excessively long so as to avoid yellowing, especially, when the temperature is relatively high. The thus obtained sheet or film of the invention is translucent and has adequate water absorptivity and biodegradability as well as sufficiently high dry and wet strength which can be controlled by selecting the amount of the chitosan and starch and the temperature of the heat treatment. The method for shaping the slurry into a form is not particularly limitative. When the desired form of the shaped article is a film or sheet, the well established technology in the paper-making process is applicable. It is a feature of the invention that, although each of the chitosan salt and gelatinised starch is soluble in water, the composite shaped article prepared from these components and the cellulose fibers is no longer disintegrable in water, presumably, as a result of the heat treatment after shaping, by which the chitosan salt is at least partly converted into free chitosan.

In the following description, examples are given to illustrate the composite material of the invention and the method for the preparation thereof in more detail although the scope of the invention is not limited thereto in any way. In the following description, the term "parts" always refers to "parts by weight".

Example 1.

An aqueous slurry was prepared by blending 100 parts of finely beaten bleached conifer pulp having a Canadian standard freeness of 10 ml or smaller with an aqueous solution of chitosan acetate derived from lobster shells in a varied amount of 1 to 100 parts as chitosan and an aqueous solution of gelatinised potato starch in an amount of 50 parts as starch. The aqueous slurry contained 0.5% by weight of the cellulose fibers. The slurry was spread over a plastic-made dish and dried and heat-treated for about 15 hours in an air-circulation oven at 70 °C so that a translucent film having a thickness of about 60 μm was obtained.

The thus prepared films of the composite material were subjected to measurement of dry and wet strength, absorption of water and evaluation of biodegradability to give the results shown in Table 1 below. The biodegradability was evaluated in an accelerated decomposition test by culturing chitosan-decomposing bacteria in the presence of the film to record the number of days taken until the film was finely disintegrated into pieces. The test of biodegradability was conducted as follows. Thus, three test pieces each 7 mm by 7 mm wide were taken in a test tube together with 5 ml of a standard liquid culture medium and a glass bead of 6 to 8 mesh fineness and, after inoculation of the culture medium with a cultured stock of a microorganism belonging to the genus of Pseudomonas isolated from a soil in Japan, the test tube was shaken at 28 °C until two of the three test pieces were disintegrated. In a rough estimation, a day in this acceleration test corresponds to 10 days of decomposition in soil though dependent on various parameters.

As is understood from the results shown in Table 1, the amount of the chitosan is a critical parameter for the wet strength of the film and a practically useful wet strength can be obtained only when the amount of the chitosan is 5% by weight or more based on the cellulose fibers.

## T a b l e 1

| Chitosan, parts | 1 | 5 | 10 | 50 | 100 |
|---|---|---|---|---|---|
| Dry strength, $kg/cm^2$ | 600 | 800 | 1000 | 1000 | 1000 |
| Wet strength, $kg/cm^2$ | < 50 | 400 | 500 | 550 | 400 |
| Water absorption, % | 250 | 200 | 150 | 80 | 50 |
| Biodegradability, days | < 1 | 2 | 3 | 4 | 20 |

Example 2.

The experimental procedure was substantially the same as in Example 1 in each of the experiments except that the amount of chitosan was always 20 parts and the amount of gelatinised potato starch was varied in the range from 10 to 500 parts per 100 parts of the cellulose pulp. The results of the tests for dry and wet strength, the amount of water absorption and the biodegradability are shown in Table 2 below.

As is understood from the results shown in Table 2, the water absorptivity is greatly influenced by the amount of the starch and adequate water absorptivity is obtained when the amount of starch is 50 parts or more per 100 parts of the cellulose pulp while an excessively large amount of starch, e.g., 500 parts, is undesirable due to the great decrease, in particular, in the wet strength of the film.

T a b l e    2

| Potato starch, parts | 10 | 50 | 100 | 300 | 500 |
|---|---|---|---|---|---|
| Dry strength, kg/cm² | 1100 | 1000 | 1000 | 450 | 400 |
| Wet strength, kg/cm² | 600 | 500 | 200 | 100 | < 50 |
| Water absorption, % | 20 | 80 | 150 | 190 | 220 |
| Biodegradability, days | 5 | 4 | 3 | 1 | 1 |

Example 3.

The experimental procedure was substantially the same as in Example 1 in each of the experiments except that the amounts of chitosan and starch were always 20 parts and 100 parts, respectively, per 100 parts of the cellulose pulp and the temperature for drying and heat treatment of the slurry spread over a metal dish was varied in the range from 30 to 200 °C. The results of the tests for dry wet strength, amount of water absorption and biodegradability of the films are shown in Table 3 below.

As is understood from the results shown in Table 3 the temperature of the heat treatment is a factor influencing the dry strength and biodegradability of the composite films. Namely, the temperature should be 50 °C or higher in order to provide a practically useful dry strength for the film without losing adequate biodegradability.

T a b l e 3

| Heat treatment at, °C | 30 | 50 | 100 | 150 | 200 |
|---|---|---|---|---|---|
| Dry strength, kg/cm$^2$ | 300 | 800 | 1000 | 1100 | 1100 |
| Wet strength, kg/cm$^2$ | 50 | 100 | 200 | 210 | 210 |
| Water absorption, % | - | 150 | 130 | 110 | 90 |
| Biodegradability, days | < 1 | 1 | 3 | 7 | 10 |

## Claims

**Claims for the following Contracting States : DE, GB, FR, IT, NL, BE**

1. A water-absorptive cellulose fiber-based composite material in the form of a shaped body which comprises:
   (A) 100 parts by weight of fine cellulose fibers having a freeness of 50 ml or less of Canadian standard freeness;
   (B) from 5 to 100 parts by weight of chitosan; and
   (C) from 10 to 300 parts by weight of gelatinised starch.

2. A method for the preparation of a cellulose fiber-based water-adsorptive composite material in the form of a shaped body which comprises the steps of:
   (a) blending 100 parts by weight of fine cellulose fibers having a freeness of 50 ml or less of Canadian standard freeness, an aqueous solution of a chitosan salt in an amount in the range from 5 to 100 parts by weight as chitosan and from 10 to 300 parts by weight of gelatinised starch to give an aqueous slurry;
   (b) shaping the aqueous slurry into a form; and
   (c) drying the thus shaped form of the aqueous slurry by heating at a temperature in the range from 50 to 200°C.

3. A method as claimed in Claim 2 wherein the chitosan salt is an acetate of chitosan.

4. A method as claimed in Claim 2 or Claim 3 wherein the degree of deacylation of the chitosan is at least 40%.

**Claims for the following Contracting State : ES**

1. A method for the preparation of a cellulose fiber-based water-adsorptive composite material in the form of a shaped body which comprises the steps of:
   (a) blending 100 parts by weight of fine cellulose fibers having a freeness of 50 ml or less of Canadian standard freeness, an aqueous solution of a chitosan salt in an amount in the range from 5 to 100 parts by weight as chitosan and from 10 to 300 parts by weight of gelatinised starch to give an aqueous slurry;
   (b) shaping the aqueous slurry into a form; and
   (c) drying the thus shaped form of the aqueous slurry by heating at a temperature in the range from 50 to 200°C.

2. A method as claimed in Claim 1 wherein the chitosan salt is an acetate of chitosan.

3. A method as claimed in Claim 1 or Claim 2 wherein the degree of deacylation of the chitosan is at least 40%.


**Patentansprüche**

**Patentansprüche für folgende Vertragsstaaten : DE, GB, FR, IT, NL, BE**

1. Zur Absorption von Wasser fähiges Verbundmaterial auf Cellulosefaserbasis in Form eines Formkörpers, umfassend
   (A) 100 Gew.-Teile feiner Cellulosefasern eines kanadischen Standardentwässerungsgrades von 50 ml oder weniger;
   (B) 5 bis 100 Gew.-Teile Chitosan und
   (C) 10 bis 300 Gew.-Teile gelatinisierte Stärke.

2. Verfahren zur Herstellung eines zur Absorption von Wasser fähigen Verbundmaterials auf Cellulosefaserbasis in Form eines Formkörpers, umfassend die folgenden Schritte:
   (a) Vermischen von 100 Gew.-Teilen feiner Cellulosefasern eines kanadischen Standardentwässerungsgrades von 50 ml oder weniger, einer wässrigen Lösung eines Chitosansalzes in einer Menge im Bereich von 5 bis 100 Gew.-Teilen in Form von Chitosan und von 10 bis 300 Gew.-Teilen gelatinisierter Stärke zur Bildung einer wässrigen Aufschlämmung;
   (b) Ausformen der wässrigen Aufschlämmung in eine Form und
   (c) Trocknen der derart ausgeformten Form einer wässrigen Aufschlämmung durch Erwärmen auf eine Temperatur im Bereich von 50 bis 200°C.

3. Verfahren nach Anspruch 2, wobei das Chitosansalz aus einem Chitosanacetat besteht.

4. 4. Verfahren nach Anspruch 2 oder Anspruch 3, wobei der Deacylierungsgrad des Chitosans mindestens 40% beträgt.

**Patentansprüche für folgenden Vertragsstaat : ES**

1. Verfahren zur Herstellung eines zur Absorption von Wasser fähigen Verbundmaterials auf Cellulosefaserbasis in Form eines Formkörpers, umfassend die folgenden Schritte:
   (a) Vermischen von 100 Gew.-Teilen feiner Cellulosefasern eines kanadischen Standardentwässerungsgrades von 50 ml oder weniger, einer wässrigen Lösung eines Chitosansalzes in einer Menge im Bereich von 5 bis 100 Gew.-Teilen in Form von Chitosan und von 10 bis 300 Gew.-Teilen gelatinisierter Stärke zur Bildung einer wässrigen Aufschlämmung;
   (b) Ausformen der wässrigen Aufschlämmung in eine Form und
   (c) Trocknen der derart ausgeformten Form einer wässrigen Aufschlämmung durch Erwärmen auf eine Temperatur im Bereich von 50 bis 200°C.

2. Verfahren nach Anspruch 1, wobei das Chitosansalz aus einem Chitosanacetat besteht.

3. Verfahren nach Anspruch 1 oder Anspruch 2, wobei der Deacylierungsgrad des Chitosans mindestens 40% beträgt.

**Revendications**

**Revendications pour les Etats contractants suivants : DE, GB, FR, IT, NL, BE**

1. Matériau composite à base de fibres de cellulose absorbant l'eau sous la forme d'un corps mis en forme qui comprend :
   (A) 100 parties en poids de fibres de cellulose fines ayant une liberté de 50 ml ou moins de liberté standard Canadien ;
   (B) de 5 à 100 parties en poids de chitosane ; et
   (C) de 10 à 300 parties en poids d'amidon gélatinisé.

2. Méthode pour la préparation d'un matériau composite absorbant l'eau à base de fibres de cellulose sous la forme d'un corps mis en forme qui comprend les étapes de :
   (a) mélanger 100 parties en poids de fibres de cellulose fines ayant une libreté de 50 ml ou moins de liberté standard Canadien, une solution aqueuse d'un sel de chitosane en une quantité dans l'intervalle de 5 à 100 parties en poids en tant que chitosane et de 10 à 300 parties en poids d'amidon gélatinisé pour donner une boue aqueuse ;
   (b) mettre en forme la boue aqueuse en une forme ; et
   (c) sécher la forme ainsi mise en forme de la boue aqueuse par chauffage à une température dans l'intervalle de 50 à 200°C.

3. Méthode selon la revendication 2 où le sel de chitosane est un acétate de chitosane.

4. Méthode selon la revendication 2 ou 3 où le degré de désacylation du chitosane est au moins de 40 %.

**Revendications pour l'Etat contractant suivant : ES**

1. Procédé pour la préparation d'un matériau composite absorbant l'eau à base de fibres de cellulose sous la forme d'un corps mis en forme qui comprend les étapes de :
   (a) mélanger 100 parties en poids de fibres de cellulose fines ayant une liberté de 50 ml ou moins de liberté standard Canadien, une solution aqueuse d'un sel de chitosane en une quantité dans l'intervalle de 5 à 100 parties en poids en tant que chitosane et de 10 à 300 parties en poids d'amidon gélatinisé pour donner une boue aqueuse ;
   (b) mettre en forme la boue aqueuse en une forme ; et
   (c) sécher la forme ainsi mise en forme de la boue aqueuse par chauffage à une température dans l'intervalle de 50 à 200°C.

2. Méthode selon la revendication 1 où le sel de chitosane est un acétate de chitosane.

3. Méthode selon la revendication 1 ou 2 où le degré de désacylation du chitosane est d'au moins 40 %.